# FASCICULE DE BREVET EUROPEEN

(11) **EP 0 856 584 B1**
(45) Date de publication et mention de la délivrance du brevet: **14.12.2005**
(21) Numéro de dépôt: 98400118.0
(22) Date de dépôt: 21.01.1998
(51) Int. Cl.: C12N 15/53, C12N 9/04, C12N 5/10, C12N 1/21, C12N 15/82, A01H 5/00, C12Q 1/68

(54) **Composés polypeptidiques à activité eutypine-réductase et séquences de nucléotides favorisant la résistance à l'eutypiose chez les végétaux**
Zusammensetzungen mit Polypeptiden die Eutypin Reductase Aktivität haben sowie Nukleotid Sequenzen die die Resistenz gegen die Eutypiose fördern
Compositions comprising polypeptides having an eutypine reductase activity and nucleotide sequences favoring the resistance against dying arm disease in plants

(30) Priorité: 29.01.1997 FR 9700962
(43) Date de publication de la demande: 05.08.1998
(73) Titulaire: Société des Domaines Viticoles Martell, 16100 Cognac (FR)
(72) Inventeur: Latche, Alain, 31076 Toulouse Cedex (FR); Roustan, Jean-Paul, 31076 Toulouse Cedex (FR); Bouzayen, Mondher, 31076 Toulouse Cedex (FR); Pech, Jean-Claude, 31076 Toulouse Cedex (FR); Fallot, Jean, 31076 Toulouse Cedex (FR)
(74) Mandataire: Desaix, Anne

(56) Documents cités:
- IUCHI S. ET AL: "Novel drought-inducible genes in the highly drought-tolerant cowpea: cloning of cDNAs and analysis of the expresseion of the corresponding genes" PLANT CELL PHYSIOLOGY, vol. 37, no. 8, décembre 1996 (1996-12), pages 1073-1082, XP002044749
- DESWARTE C. ET AL: "Transport, cytoplasmic accumulation and mechanism of action of the toxin eutypine in Vitis vinifera cells" J. PLANT PHYSIOL., vol. 149, 1996, pages 336-342, XP002044750
- DESWARTE C. ET AL: "Protonophoric activity of eutypine, a toxin from Eutypa lata, in plant mitochondria" ARCHIVES OF BIOCHEMISTRY AND BIOPHYSICS, vol. 334, octobre 1996 (1996-10), pages 200-205, XP002044751
- GUILLEN PEDRO ET AL: "A novel NADPH-dependent aldehyde reductase gene from Vigna radiata confers resistance to the grapevine fungal toxin eutypine." PLANT JOURNAL NOV., 1998, vol. 16, no. 3, novembre 1998 (1998-11), pages 335-343, XP000914749 ISSN: 0960-7412

### LISTE DE SEQUENCES

(1) INFORMATIONS GENERALES:
   (i) DEPOSANT:
      (A) NOM: SOCIETE DES DOMAINES VITICOLES MARTELL
      (B) RUE: 7, Place Edouard Martell
      (C) VILLE: COGNAC
      (E) PAYS: FRANCE
      (F) CODE POSTAL: 16100
   (ii) TITRE DE L' INVENTION: COMPOSES POLYPEPTIDIQUES ET SEQUENCES DE NUCLEOTIDES FAVORISANT LA RESISTANCE A L'EUTYPIOSE CHEZ LES VEGETAUX
   (iii) NOMBRE DE SEQUENCES: 4
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (v) DONNEES DE LA DEMANDE ACTUELLE: NUMERO DE LA DEMANDE: EP 98400118.0
   (vi) DONNEES DE LA DEMANDE ANTERIEURE:
      (A) NUMERO DE LA DEMANDE: FR 9700962
      (B) DATE DE DEPOT: 29-JAN-1998
(2) INFORMATIONS POUR LA SEQ ID NO: 1:
   (i) CARACTERISTIQUES DE LA SEQUENCE:
      (A) LONGUEUR: 975 paires de bases
      (B) TYPE: nucléotide
      (C) NOMBRE DE BRINS: double
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:1..975
      (D) AUTRES INFORMATIONS:/note= "SEQUENCE POLYPEPTIDIQUE A ACTIVITE EUTYPINE-REDUCTASE DEDUITE DE LA SEQUENCE DE NUCLEOTIDES"
   (xi) DESCRIPTION DE LA SEQUENCE: SEQ ID NO: 1:
   ATG AGC ACC GCC GCT GGA AAA CTA GTC TGT GTC ACC GGC GCT TCC GGT        48
   Met Ser Thr Ala Ala Gly Lys Leu Val Cys Val Thr Gly Ala Ser Gly
     1               5                  10                  15
   TAC ATC GCT TCC TGG CTC GTC AAG TTT CTT CTC GAA CGC GGC TAC ACC      96
   Tyr Ile Ala Ser Trp Leu Val Lys Phe Leu Leu Glu Arg Gly Tyr Thr
               20                  25                  30
   GTG AAG GCC ACC GTT CGC GAC ACG AAT GAT CCC AAA AAG GTA GAT CAC     144
   Val Lys Ala Thr Val Arg Asp Thr Asn Asp Pro Lys Lys Val Asp His
           35                  40                  45
   TTG CTC AGC CTT GAT GGT GCG AAG GAG AGA TTG CAT CTC GTG AAG GCG     192
   Leu Leu Ser Leu Asp Gly Ala Lys Glu Arg Leu His Leu Val Lys Ala
       50                  55                  60
   AAT CTT CTA GAA GAA GGT TCC TTT GAC TCT GCC GTT GAA GGT GTT CAC     240
   Asn Leu Leu Glu Glu Gly Ser Phe Asp Ser Ala Val Glu Gly Val His
   65                  70                  75                  80
   GCT GTG TTT CAC ACT GCT TCT CCC TTT TTC AAC GAT GCC AAA GAT CCG     288
   Ala Val Phe His Thr Ala Ser Pro Phe Phe Asn Asp Ala Lys Asp Pro
                   85                  90                  95
   CAG ACT GAG TTG TTG GAC CCG GCA GTG AAG GGG ACT CTG AAT GTT CTG     336
   Gln Thr Glu Leu Leu Asp Pro Ala Val Lys Gly Thr Leu Asn Val Leu
               100                 105                 110
   AAA TCG TGT GTG AAC TCG CCC ACG CTG AAA CGC GTC GTT TTA ACT TCT     384
   Lys Ser Cys Val Asn Ser Pro Thr Leu Lys Arg Val Val Leu Thr Ser
           115                 120                 125
   TCT ATT GCT GCA GTT GCG TTC AGC GAC AGG CCT AAA AAC CCT GAT GTT     432
   Ser Ile Ala Ala Val Ala Phe Ser Asp Arg Pro Lys Asn Pro Asp Val
       130                 135                 140
   GTG GTT GAC GAG ACT TGG TAT TCT GAC CCG GAA TAC TGT AAG AGA ACA     480
   Val Val Asp Glu Thr Trp Tyr Ser Asp Pro Glu Tyr Cys Lys Arg Thr
   145                 150                 155                 160
   GGG TTA TGG TAT AAC CTT TCA AAG ACT CTG GCC GAA GAT GCT GCC TGG     528
   Gly Leu Trp Tyr Asn Leu Ser Lys Thr Leu Ala Glu Asp Ala Ala Trp
                   165                 170                 175
   AAA TTT GCG AAA GAA AAC AAC ATT GAC CTG GTT ACA ATG AAC CCA GCA     576
   Lys Phe Ala Lys Glu Asn Asn Ile Asp Leu Val Thr Met Asn Pro Ala
               180                 185                 190
   TTG GTT GTT GGA CCT CTC TTG CAA CCA GTG CTT AAT ACT AGC GCT GCT     624
   Leu Val Val Gly Pro Leu Leu Gln Pro Val Leu Asn Thr Ser Ala Ala
           195                 200                 205
   ATA GTT TTA GGT TTA GTT AAT GGG GCG AAA ACA TTT AAA AAT GCT TCT     672
   Ile Val Leu Gly Leu Val Asn Gly Ala Lys Thr Phe Lys Asn Ala Ser
       210                 215                 220
   TTG GGA TGG GTC GAC GTG AAA GAT GTT GCA TTG GCC CAT ATT CTA GCA     720
   Leu Gly Trp Val Asp Val Lys Asp Val Ala Leu Ala His Ile Leu Ala
   225                 230                 235                 240
   TAT GAG AAT GCT TCG GCT AAT GGA AGA TAT TTA CTA GTT GAG AGA GTA    768
   Tyr Glu Asn Ala Ser Ala Asn Gly Arg Tyr Leu Leu Val Glu Arg Val
                   245                 250                 255
   GCA CAC TTT GGA GAT GCT GCC AAG ATT TTA CGA GAT TTA TAC CCA ACA    816
   Ala His Phe Gly Asp Ala Ala Lys Ile Leu Arg Asp Leu Tyr Pro Thr
               260                 265                 270
   TTG CAA ATT CCA GAC AAG TGT GAA GAC GAT AAG CCA TTA GAA CCA ATA    864
   Leu Gln Ile Pro Asp Lys Cys Glu Asp Asp Lys Pro Leu Glu Pro Ile
           275                 280                 285
   TTT CAG GTT TCG AAG GAA AAA GCA AAG AGC TTG GGG ATT GAC TAT ATT    912
   Phe Gln Val Ser Lys Glu Lys Ala Lys Ser Leu Gly Ile Asp Tyr Ile
       290                 295                 300
   CCT TTG GAA GTG AGC CTC AAG GAC ACT GTG GAG AGC TTG AAG GAA AAG    960
   Pro Leu Glu Val Ser Leu Lys Asp Thr Val Glu Ser Leu Lys Glu Lys
   305                 310                 315                 320
   AAG TTT CTG AAA GTT                                                975
   Lys Phe Leu Lys Val
                   325
(2) INFORMATIONS POUR LA SEQ ID NO: 2:
   (i) CARACTERISTIQUES DE LA SEQUENCE:
      (A) LONGUEUR: 325 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Ser Thr Ala Ala Gly Lys Leu Val Cys Val Thr Gly Ala Ser Gly
   1               5                   10                  15
   Tyr Ile Ala Ser Trp Leu Val Lys Phe Leu Leu Glu Arg Gly Tyr Thr
               20                  25                  30
   Val Lys Ala Thr Val Arg Asp Thr Asn Asp Pro Lys Lys Val Asp His
           35                  40                  45
   Leu Leu Ser Leu Asp Gly Ala Lys Glu Arg Leu His Leu Val Lys Ala
       50                  55                  60
   Asn Leu Leu Glu Glu Gly Ser Phe Asp Ser Ala Val Glu Gly Val His
   65                  70                  75                  80
   Ala Val Phe His Thr Ala Ser Pro Phe Phe Asn Asp Ala Lys Asp Pro
                   85                  90                  95
   Gln Thr Glu Leu Leu Asp Pro Ala Val Lys Gly Thr Leu Asn Val Leu
               100                 105                 110
   Lys Ser Cys Val Asn Ser Pro Thr Leu Lys Arg Val Val Leu Thr Ser
           115                 120                 125
   Ser Ile Ala Ala Val Ala Phe Ser Asp Arg Pro Lys Asn Pro Asp Val
       130                 135                 140
   Val Val Asp Glu Thr Trp Tyr Ser Asp Pro Glu Tyr Cys Lys Arg Thr
   145                 150                 155                 160
   Gly Leu Trp Tyr Asn Leu Ser Lys Thr Leu Ala Glu Asp Ala Ala Trp
                   165                 170                 175
   Lys Phe Ala Lys Glu Asn Asn Ile Asp Leu Val Thr Met Asn Pro Ala
               180                 185                 190
   Leu Val Val Gly Pro Leu Leu Gln Pro Val Leu Asn Thr Ser Ala Ala
           195                 200                 205
   Ile Val Leu Gly Leu Val Asn Gly Ala Lys Thr Phe Lys Asn Ala Ser
       210                 215                 220
   Leu Gly Trp Val Asp Val Lys Asp Val Ala Leu Ala His Ile Leu Ala
   225                 230                 235                 240
   Tyr Glu Asn Ala Ser Ala Asn Gly Arg Tyr Leu Leu Val Glu Arg Val
                   245                 250                 255
   Ala His Phe Gly Asp Ala Ala Lys Ile Leu Arg Asp Leu Tyr Pro Thr
               260                 265                 270
   Leu Gln Ile Pro Asp Lys Cys Glu Asp Asp Lys Pro Leu Glu Pro Ile
           275                 280                 285
   Phe Gln Val Ser Lys Glu Lys Ala Lys Ser Leu Gly Ile Asp Tyr Ile
       290                 295                 300
   Pro Leu Glu Val Ser Leu Lys Asp Thr Val Glu Ser Leu Lys Glu Lys
   305                 310                 315                 320
   Lys Phe Leu Lys Val
                   325
(2) INFORMATIONS POUR LA SEQ ID NO: 3:
   (i) CARACTERISTIQUES DE LA SEQUENCE:
      (A) LONGUEUR: 1254 paires de bases
      (B) TYPE: nucléotide
      (C) NOMBRE DE BRINS: double
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:22..996
      (D) AUTRES INFORMATIONS:/note= "SEQUENCE PEPTIDIQUE DEDUITE DE LA SEQUENCE NUCLEOTIDIQUE DE L'ADNc PLEINE LONGUEUR DU GENE VRER"
   (xi) DESCRIPTION DE LA SEQUENCE: SEQ ID NO: 3:
   GCAGAATCAA CATAATCCAC A ATG AGC ACC GCC GCT GGA AAA CTA GTC TGT          51
                           Met Ser Thr Ala Ala Gly Lys Leu Val Cys
                                               330                 335
   GTC ACC GGC GCT TCC GGT TAC ATC GCT TCC TGG CTC GTC AAG TTT CTT          99
   Val Thr Gly Ala Ser Gly Tyr Ile Ala Ser Trp Leu Val Lys Phe Leu
                   340                 345                 350
   CTC GAA CGC GGC TAC ACC GTG AAG GCC ACC GTT CGC GAC ACG AAT GAT         147
   Leu Glu Arg Gly Tyr Thr Val Lys Ala Thr Val Arg Asp Thr Asn Asp
               355                 360                 365
   CCC AAA AAG GTA GAT CAC TTG CTC AGC CTT GAT GGT GCG AAG GAG AGA         195
   Pro Lys Lys Val Asp His Leu Leu Ser Leu Asp Gly Ala Lys Glu Arg
           370                 375                 380
   TTG CAT CTC GTG AAG GCG AAT CTT CTA GAA GAA GGT TCC TTT GAC TCT         243
   Leu His Leu Val Lys Ala Asn Leu Leu Glu Glu Gly Ser Phe Asp Ser
       385                 390                 395
   GCC GTT GAA GGT GTT CAC GCT GTG TTT CAC ACT GCT TCT CCC TTT TTC         291
   Ala Val Glu Gly Val His Ala Val Phe His Thr Ala Ser Pro Phe Phe
   400                 405                 410                 415
   AAC GAT GCC AAA GAT CCG CAG ACT GAG TTG TTG GAC CCG GCA GTG AAG         339
   Asn Asp Ala Lys Asp Pro Gln Thr Glu Leu Leu Asp Pro Ala Val Lys
                   420                 425                 430
   GGG ACT CTG AAT GTT CTG AAA TCG TGT GTG AAC TCG CCC ACG CTG AAA         387
   Gly Thr Leu Asn Val Leu Lys Ser Cys Val Asn Ser Pro Thr Leu Lys
               435                 440                 445
   CGC GTC GTT TTA ACT TCT TCT ATT GCT GCA GTT GCG TTC AGC GAC AGG         435
   Arg Val Val Leu Thr Ser Ser Ile Ala Ala Val Ala Phe Ser Asp Arg
           450                 455                 460
   CCT AAA AAC CCT GAT GTT GTG GTT GAC GAG ACT TGG TAT TCT GAC CCG 483
   Pro Lys Asn Pro Asp Val Val Val Asp Glu Thr Trp Tyr Ser Asp Pro
   465 470 475
   GAA TAC TGT AAG AGA ACA GGG TTA TGG TAT AAC CTT TCA AAG ACT CTG 531
   Glu Tyr Cys Lys Arg Thr Gly Leu Trp Tyr Asn Leu Ser Lys Thr Leu
   480 485 490 495
   GCC GAA GAT GCT GCC TGG AAA TTT GCG AAA GAA AAC AAC ATT GAC CTG 579
   Ala Glu Asp Ala Ala Trp Lys Phe Ala Lys Glu Asn Asn Ile Asp Leu
   500 505 510
   GTT ACA ATG AAC CCA GCA TTG GTT GTT GGA CCT CTC TTG CAA CCA GTG 627
   Val Thr Met Asn Pro Ala Leu Val Val Gly Pro Leu Leu Gln Pro Val
   515 520 525
   CTT AAT ACT AGC GCT GCT ATA GTT TTA GGT TTA GTT AAT GGG GCG AAA 675
   Leu Asn Thr Ser Ala Ala Ile Val Leu Gly Leu Val Asn Gly Ala Lys
   530 535 540
   ACA TTT AAA AAT GCT TCT TTG GGA TGG GTC GAC GTG AAA GAT GTT GCA 723
   Thr Phe Lys Asn Ala Ser Leu Gly Trp Val Asp Val Lys Asp Val Ala
   545 550 555
   TTG GCC CAT ATT CTA GCA TAT GAG AAT GCT TCG GCT AAT GGA AGA TAT 771
   Leu Ala His Ile Leu Ala Tyr Glu Asn Ala Ser Ala Asn Gly Arg Tyr
   560 565 570 575
   TTA CTA GTT GAG AGA GTA GCA CAC TTT GGA GAT GCT GCC AAG ATT TTA 819
   Leu Leu Val Glu Arg Val Ala His Phe Gly Asp Ala Ala Lys Ile Leu
   580 585 590
   CGA GAT TTA TAC CCA ACA TTG CAA ATT CCA GAC AAG TGT GAA GAC GAT 867
   Arg Asp Leu Tyr Pro Thr Leu Gln Ile Pro Asp Lys Cys Glu Asp Asp
   595 600 605
   AAG CCA TTA GAA CCA ATA TTT CAG GTT TCG AAG GAA AAA GCA AAG AGC 915
   Lys Pro Leu Glu Pro Ile Phe Gln Val Ser Lys Glu Lys Ala Lys Ser
   610 615 620
   TTG GGG ATT GAC TAT ATT CCT TTG GAA GTG AGC CTC AAG GAC ACT GTG 963
   Leu Gly Ile Asp Tyr Ile Pro Leu Glu Val Ser Leu Lys Asp Thr Val
   625 630 635
   GAG AGC TTG AAG GAA AAG AAG TTT CTG AAA GTT TAATGTTACC CTTCAAGAAA 1016
   Glu Ser Leu Lys Glu Lys Lys Phe Leu Lys Val
   640 645 650
   CGTGGAAACC TTCTTATGCC TATGCTTGAG GTAAACTTAG TGTTAAATAA ATGGAAAGTG 1076
   ATGTAAGTTC TGTAATAAGT GATTCTTGGA CTGGTTTTTT CTGGTGACAA GTTTCAAAAA 1136
   TGAGTGACCA TGGTAGAGTC TGCGTTCCAG ATTGTATCCT TGTGATATTG TATTAGTAGA 1196
   AGATGTTTGT TGCAGCTAAT GCACCCATTA TTATGTTTTT ATCAAAAAAA AAAAAAAA 1254
(2) INFORMATIONS POUR LA SEQ ID NO: 4:
   (i) CARACTERISTIQUES DE LA SEQUENCE:
      (A) LONGUEUR: 325 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Ser Thr Ala Ala Gly Lys Leu Val Cys Val Thr Gly Ala Ser Gly
     1               5                  10                  15
   Tyr Ile Ala Ser Trp Leu Val Lys Phe Leu Leu Glu Arg Gly Tyr Thr
                20                  25                  30
   Val Lys Ala Thr Val Arg Asp Thr Asn Asp Pro Lys Lys Val Asp His
            35                  40                  45
   Leu Leu Ser Leu Asp Gly Ala Lys Glu Arg Leu His Leu Val Lys Ala
        50                  55                  60
   Asn Leu Leu Glu Glu Gly Ser Phe Asp Ser Ala Val Glu Gly Val His
    65                  70                  75                  80
   Ala Val Phe His Thr Ala Ser Pro Phe Phe Asn Asp Ala Lys Asp Pro
                    85                  90                  95
   Gln Thr Glu Leu Leu Asp Pro Ala Val Lys Gly Thr Leu Asn Val Leu
               100                 105                 110
   Lys Ser Cys Val Asn Ser Pro Thr Leu Lys Arg Val Val Leu Thr Ser
           115                 120                 125
   Ser Ile Ala Ala Val Ala Phe Ser Asp Arg Pro Lys Asn Pro Asp Val
       130                 135                 140
   Val Val Asp Glu Thr Trp Tyr Ser Asp Pro Glu Tyr Cys Lys Arg Thr
   145                 150                 155                 160
   Gly Leu Trp Tyr Asn Leu Ser Lys Thr Leu Ala Glu Asp Ala Ala Trp
                   165                 170                 175
   Lys Phe Ala Lys Glu Asn Asn Ile Asp Leu Val Thr Met Asn Pro Ala
               180                 185                 190
   Leu Val Val Gly Pro Leu Leu Gln Pro Val Leu Asn Thr Ser Ala Ala
           195                 200                 205
   Ile Val Leu Gly Leu Val Asn Gly Ala Lys Thr Phe Lys Asn Ala Ser
       210                 215                 220
   Leu Gly Trp Val Asp Val Lys Asp Val Ala Leu Ala His Ile Leu Ala
   225                 230                 235                 240
   Tyr Glu Asn Ala Ser Ala Asn Gly Arg Tyr Leu Leu Val Glu Arg Val
                   245                 250                 255
   Ala His Phe Gly Asp Ala Ala Lys Ile Leu Arg Asp Leu Tyr Pro Thr
               260                 265                 270
   Leu Gln Ile Pro Asp Lys Cys Glu Asp Asp Lys Pro Leu Glu Pro Ile
           275                 280                 285
   Phe Gln Val Ser Lys Glu Lys Ala Lys Ser Leu Gly Ile Asp Tyr Ile
       290                 295                 300
   Pro Leu Glu Val Ser Leu Lys Asp Thr Val Glu Ser Leu Lys Glu Lys
   305                 310                 315                 320
   Lys Phe Leu Lys Val
                   325

## Description

L'invention a pour objet des composés intervenant dans la résistance à l'eutypiose chez les végétaux, ainsi que des moyens de lutte contre cette maladie, par exemple chez la vigne.

L'eutypiose est actuellement la plus grave maladie de la vigne. En effet, elle est présente dans les vignobles du monde entier ; elle conduit à la mort prématurée de nombreux ceps chez les variétés sensibles et il n'existe pas de moyens de lutte curatifs. Le parasite responsable de l'eutypiose de la vigne est un champignon ascomycète, existant sous une forme parfaite *Eutypa lata* (Carter M.V., 1955, Apricot gummosis - a new development. J. Dep. Agric. South Aust. **59**:178-184) Tul. et C. Tul. Syn. *E armeniacae* Hansf. et Carter) ou imparfaite *Libertella blepharis* (Samuel G., 1933 « Gummosis » or « Dieback » in apricot trees. J. Dep. Agric. South. Aust. **36**:979-980) A.L. Smith (syn. Cytosporina sp.).

L'eutypiose est certainement une maladie ancienne, même si l'agent pathogène n'a été observé sur la vigne qu'en 1973 en Australie. En France, la maladie fut identifiée avec certitude par Bolay en 1977 dans la région du Languedoc-Roussillon. A partir de 1978, les observations se multiplièrent dans le vignoble français, montrant l'extension de l'eutypiose. Les raisons du développement de cette maladie de dépérissement, qui existait à l'état endémique, ne sont pas clairement identifiées aujourd'hui ; selon certains auteurs, cette progression pourrait être imputée à la modification de certaines pratiques culturales.

La vigne n'est pas la seule plante ligneuse pérenne hôte de ce parasite. En effet, les manifestations de l'eutypiose ont été observées notamment sur l'abricotier, le cassissier, le cerisier, le tamaris, l'amandier, le pommier.

La maladie affecte le tronc et les "bras" de la souche de vigne, lieux de développement du mycélium, et les parties herbacées qui cependant n'hébergent jamais le parasite. Les ascopores du champignon, libérées par les asques des périthèces situés sur les écorces des ceps contaminés, pénètrent par les plaies résultant de la taille, dans la lumière des vaisseaux du xylème puis germent à quelques millimètres de la surface de la blessure et colonisent le xylème, le cambium et le phloème. L'installation du mycélium dans le "bois" du tronc et les bras des souches conduit à la formation d'une nécrose brune, dure et sectorielle, toujours bien délimitée Le mycélium actif est localisé en bordure de la nécrose, qui présente un aspect de pourriture sèche, signe d'une dégradation préférentielle de la cellulose du bois.

Les plantes atteintes d'eutypiose montrent au printemps des symptômes typiques. Dans les cas les plus caractéristiques, les rameaux sont chétifs avec des entre-noeuds courts ; les feuilles de petites taille, souvent enroulées ou même déformées, présentent un aspect légèrement chlorotique et parfois de petites nécroses marginales. Les inflorescences semblent se développer normalement jusqu'à la floraison, mais elles peuvent ensuite se nécroser ou donner des grappes pourvues de petites baies (millerandage). Les symptômes décrits affectent souvent un bras mais la maladie peut ensuite s'étendre à l'ensemble de la souche, conduisant à la mort de celle-ci.

La durée de la lente incubation du champignon dans le tronc et les bras (3 à 10 ans) confère à cette maladie un caractère insidieux. L'expression des symptômes sur les organes herbacés est aléatoire selon les années : un cep contaminé ne présente pas régulièrement de symptômes, certainement pour des raisons liées aux facteurs de l'environnement et notamment aux conditions climatiques. Par ailleurs, l'existence d'isolats d'agressivité différente a été démontrée rendant encore plus difficile la perception de la maladie.

La destruction du parasite dans le tronc des ceps ou la réduction de son expression n'est pas possible aujourd'hui. En l'absence de moyens de lutte curative, différentes mesures prophylactiques et préventives ont été conseillées pour contenir la maladie. Il est recommandé par exemple de limiter la pression de l'inoculum en éliminant les vieilles souches et les bras sectionnés après l'opération de la taille. La connaissance des conditions de libération et de germination des ascospores a conduit à plusieurs préconisations : tailler tardivement en tenant compte des conditions climatiques (temps sec et calme), éviter les tailles provoquant des plaies de grande dimension, protéger chaque plaie de taille par l'application manuelle d'un fongicide, pour limiter la germination des ascospores. Enfin, les souches atteintes peuvent être restaurées par l'opération du "recépage", consistant à conserver une tige née en dessous de la partie malade du tronc et à éliminer la partie supérieure.

On voit que l'eutypiose a une nuisibilité certaine puisque les moyens actuels de lutte, uniquement prophylactique et préventive, sont contraignants, coûteux et de portée limitée.

L'impact économique de cette maladie est important. Les enquêtes effectuées depuis 1988 en France montrent que les taux de symptômes sont variables selon les cépages et, pour un même cépage, selon les années et les régions, allant parfois jusqu'à 50% de ceps porteurs de la maladie. Parmi les variétés les plus sensibles, on trouve le Sauvignon blanc, le Cabernet Sauvignon, l'Ugni blanc, le Cinsault et le Chein alors que le Merlot et le Sémillon sont les plus tolérants.

Un réseau d'observations, fondé sur une approche statistique rigoureuse, mis en place dans le cadre d'un contrat européen (Programme "Lutte contre l'eutypiose", n° 8001-CT-91.205) a porté sur 85000 ceps cultivés dans 11 régions viticoles et sur 10 cépages Il a permis de montrer que tous les cépages présentent des symptômes d'eutypiose, mais avec des taux très variables : 30 à 80 % dans certaines régions de Grèce, 5 à 10 % dans la région de Rioja Alta (Espagne, cépage Tempranillo) et en Italie (cépage Trebbiano), et inférieur à 2 % voire 1 % dans les régions viticoles du Portugal.

Cette maladie peut aussi entraîner une réduction de l'expression aromatique chez certain cépage. En outre, l'eutypiose exige le remplacement prématuré des souches mortes, ce qui provoque le rajeunissement du vignoble préjudiciable à la qualité. II est à noter encore que le champignon parasite joue un rôle de pionnier pour l'installation de l'esca (Carignon C., 1991), complexe fongique responsable également d'un déperissement de la vigne.

Cette maladie a aussi des effets indirects sur la qualité en modifiant l'encépagement des vignobles puisque les variétés sensibles sont peu à peu remplacées par des variétés plus tolérantes, dans le respect toutefois des contraintes des appellations d'origine. De plus, si le volume de la récolte est maintenu malgré un taux de souches manquantes élevé, la qualité des vins peut être affectée.

En conséquence, il est clair que pour les viticulteurs souhaitant maintenir une production régulière de vins de qualité, l'eutypiose est actuellement la maladie la plus préoccupante.

Les inventeurs ont montré que l'eutypiose est associée à la synthèse d'un composé de la famille des acides lipophiles, l'hydroxy-4(methyl-3-butène-3-ynyl-1)-3 benzaldéhyde, appelé eutypine (Tey Rulh P. et al. Phytochemistry, vol. 30, n° 2, pp. 471-473, 1991), dans certains tissus de l'hôte hébergeant le champignon parasite. Cette molécule toxique est synthétisée par le champignon parasite.

Afin de lutter contre cette maladie, les inventeurs se sont intéressés à la structure et à la physiologie de l'eutypine lorsqu'elle est synthétisée chez l'hôte par le parasite et ont observé chez les plantes et en particulier chez les plantes résistantes à l'eutypiose, la synthèse de produits de dégradation de ce constituant toxique.

Par l'expression « plantes résistantes à l'eutypiose », on entend dans le cadre de la présente demande, la capacité des plantes contaminées par le parasite responsable, à résister aux effets nuisibles induits directement ou indirectement par la présence du parasite

Ce type de résistance peut encore être expliqué comme la capacité des plantes contaminées par le champignon responsable de l'eutypiose, à tolérer la présence de ce parasite sans que leur développement, y compris leur croissance et leur physiologie, soit affecté dans une mesure qui entraînerait le dépérissement des plantes en question.

Les inventeurs ont mis en évidence une voie de métabolisation de l'eutypine au sein des végétaux contaminés par le parasite, aboutissant à la synthèse de produits non toxiques pour ces végétaux ; ils ont proposé, sur cette base, des moyens pour conférer à des plantes sensibles à l'eutypiose, des propriétés de résistance ou le cas échéant des moyens pour augmenter les capacités de résistance déjà existantes chez certains végétaux, vis à vis de l'eutypiose.

A cet égard, les inventeurs ont notamment identifié et caractérisé un polypeptide à activité enzymatique de type réductase, capable de favoriser la dégradation de l'eutypine toxique, en constituants non toxiques incluant un composé appelé eutypinol.

Les inventeurs ont isolé et identifié l'enzyme eutypine-réductase et sont parvenus à caractériser la séquence nucléotidique codant pour cette enzyme.

La séquence de nucléotides codant pour cette enzyme constitue donc un moyen nouveau pour envisager la production de plantes résistantes à l'eutypiose conformément à la définition qui a été donnée précédemment de cette résistance. Par exemple, la caractérisation de cette séquence de nucléotides permet de préparer des plantes transgéniques susceptibles de surexprimer l'eutypine-réductase et d'augmenter la métabolisation de l'eutypine produite chez l'hôte par ie parasite.

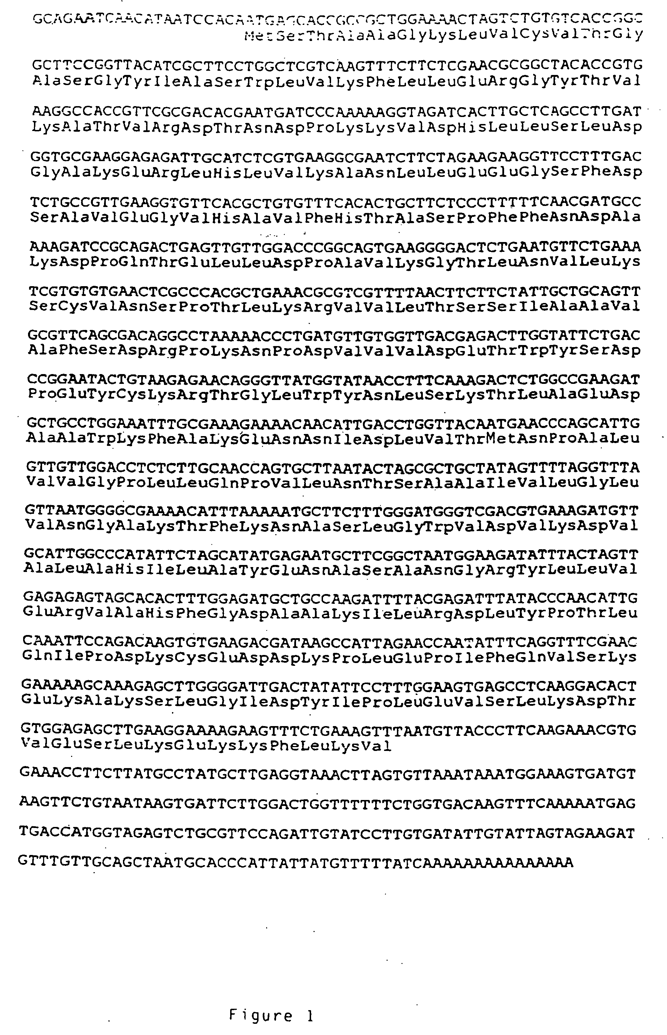

L'invention a donc pour objet une séquence de nucléotides, caractérisée en ce qu'elle code pour un polypeptide à activité eutypine-réductase, ce polypeptide comprenant la séquence d'acides aminés représentée à la figure 1.

Selon un mode de réalisation particulier de l'invention, cette séquence de nucléotides est caractérisée en ce qu'elle comprend l'enchaînement de nucléotides compris entre les nucléotides 22 et 975 de la séquence de nucléotides représentée à la figure 1.

En variante, une telle séquence est caractérisée en ce qu'elle correspond à l'enchaînement représenté à la figure 1, qui est une séquence d'ADN complémentaire (ADNc) du gène VRER codant pour la molécule eutypine-réductase.

L'invention porte également sur une séquence de nucléotides caractérisée en ce qu'elle code pour un polypeptide à activité eutypine-réductase et en ce qu'elle présente une similitude du nombre de nucléotides supérieure ou égale à 90%, avec l'une des séquences précédemment décrite.

La présente invention a également pour objet une séquence de nucléotides hybridant dans des conditions de forte stringence avec l'une des séquences précédemment décrites.

De telles conditions de forte stringence sont les suivantes : mise en contact avec une solution contenant SSC (2X), 1% SDS pendant 5 min à 25°C, puis deux fois 15 min à 45°C puis dans une solution contenant SSC (0,2X), 1% SDS deux fois pendant 15 min à 45°C.

Alternativement, la séquence de nucléotides de l'invention peut être définie en ce qu'elle présente une similitude en nombre de nucléotides, supérieure à 75% avec la séquence de nucléotides comprise entre les nucléotides 22 et 975 de l'enchaînement de la figure 1, ou avec la séquence de nucléotides de la figure 1 et en ce qu'elle code pour un polypeptide à activité eutypine-réductase et en ce qu'elle est placée sous le contrôle d'un promoteur approprié pour l'expression hétérologue.

De préférence, cette similitude est supérieure ou égale à 80% et avantageusement supérieure ou égale à 90%.

Les séquences de nucléotides peuvent avantageusement être utilisées dans des techniques de génie génétique pour produire les composés selon l'invention. En particulier, les séquences de nucléotides selon l'invention sont contenues dans un vecteur d'expression et/ou de clonage, par exemple le vecteur binaire pGA.

A cet égard, on utilisera avantageusement une séquence répondant à l'une des définitions précédentes sous le contrôle d'un promoteur homologue ou hétérologue, et de préférence, sous le contrôle d'un promoteur approprié pour l'expression dans des cellules procaryotes et/ou eucaryotes.

De préférence, et selon le niveau de l'activité eutypine-réductase recherché, on aura recours à un promoteur fort pour exprimer une séquence de nucléotides selon l'invention Un promoteur approprié est par exemple le promoteur fort 35S du CaMV (EP-B-0131623) permettant l'expression dans des cellules végétales.

Le cas échéant, le promoteur utilisé est associé à d'autres séquences de régulation telles que des activateurs d'expression.

A titre d'exemple de vecteurs, on citera le vecteur binaire pGA décrit par An, G. et al, 1988, Binary Vectors, pp.1-19 in Plant Molecular Biology Manual A3 Kluwer Acad. Pub., Dordrecht, The Netherlands.

La demande concerne également des cellules recombinantes procaryotes ou eucaryotes, contenant une séquence de nucléotides répondant à l'une des définitions données ci-dessus.

Des cellules recombinantes intéressantes dans le cadre de la réalisation de l'invention sont par exemple des cellules bactériennes, notamment des cellules de E. coli ou d'Agrobacterium tumefaciens. Il peut également s'agir de cellules eucaryotes et en particulier de cellules végétales. A titre d'exemple, on aura recours à des cellules de semence de plante ou à des cellules de vigne, notamment lorsque l'on souhaite mettre en oeuvre chez la vigne la résistance à l'eutypiose.

Entre également dans le cadre de l'invention, un tissu biologique ou une plante produisant un composé polypeptidique répondant à l'une des définitions précédentes après transformation par une séquence de nucléotides selon l'invention. De préférence, la plante recombinante est la vigne

L'invention se rapporte également à des semences obtenues à partir desdites plantes ainsi qu'à un procédé de production de ces plantes ou de ces semences.

De préférence, les cellules, tissus et plantes transformés dans le cadre de l'invention contiennent la séquence de nucléotides intégrée de façon stable dans leur génome. Toute technique de transformation appropriée peut être mise en oeuvre, telle que l'électroporation, le bombardement de microprojectiles portant de l'ADN, par exemple au moyen d'un canon à particules, la co-culture d'explants en présence d'Agrobacterium tumefaciens.

Un tel procédé de production de plante ou de semence exprimant un composé polypeptidique à activité eutypine-réductase est caractérisé en ce qu'il comprend les étapes de
a) transformation d'une cellule végétale, avec une séquence de nucléotides définie ci-dessus ou un vecteur défini ci-dessus, dans des conditions permettant l'expression de façon stable et fonctionnelle de la protéine à activité eutypine-réductase codée par la susdite séquence de nucléotides;
b) régénération de plantes à partir de la cellule de plante transformée de l'étape a), pour obtenir des plantes exprimant la protéine à activité eutypine-réductase,
c) le cas échéant, obtention de semences à partir des plantes modifiées obtenues à l'étape b).

Le procédé de l'invention peut avantageusement être mis en oeuvre pour produire des plantes de la famille de la vigne, mais également pour produire des plantes résistantes à l'eutypiose lorsque ces plantes sont naturellement sensibles à cette maladie, par exemple des plantes ligneuses pérennes telles que l'abricotier, le cassissier, le cerisier, le tamaris, l'amandier ou le pommier.

L'invention a par ailleurs pour objet l'utilisation de la séquence de nucléotides telle que définie dans les pages précédentes, en tant qu'agent de sélection révélateur de la transformation de cellules par une séquence de nucléotides déterminée dont l'expression est couplée à celle de la séquence utilisée comme agent de sélection: Dans le cadre de cette application, la séquence ou une partie de la séquence du gène VRER ou de son ADNc peut être utilisée comme gène de sélection pour permettre d'identifier des cellules, des tissus ou des plantes transformés génétiquement par une séquence d'intérêt. Dans ce cas, le gène de sélection est transféré avec le gène d'intérêt au sein des cellules que l'on cherche à transformer. La séquence du gène d'intérêt et celle du gène de sélection sont associées de façon telle que l'intégration et/ou l'expression de l'une est liée à l'intégration et/ou l'expression de l'autre. L'intérêt de l'utilisation de la séquence de l'invention comme gène ou séquence de sélection permet notamment d'éviter certains problèmes liés à la dissémination des gènes, problème rencontré avec les gènes de résistance aux antibiotiques ou aux herbicides. Dans le cadre de cette application, les cellules, tissus ou plantes transformés peuvent être sélectionnés par mise en contact avec l'eutypine ou avec tout substrat susceptible d'être réduit par l'eutypine-réductase produite par le gène de sélection.

La sélection des cellules, tissus ou organismes transformés ayant intégré le gène de sélection, à l'aide de l'eutypine ou de tout substrat approprié, pourra être faite en déterminant auparavant le seuil de sensibilité à l'eutypine de la cellule du tissu ou de la plante considérés. Cette détermination sera faite par des techniques analogues à celles connues pour la détermination de la sensibilité d'une espèce ou d'un organisme donné à un antibiotique ou à un herbicide.

L'invention a donc pour objet également un acide nucléique recombinant contenant une séquence de nucléotides selon l'invention, utilisée comme agent de sélection en association avec une séquence d'intérêt, ladite association permettant l'intégration conjointe du marqueur de sélection et de la séquence d'intérêt, dans un hôte cellulaire déterminé.

Entre aussi dans le cadre de l'invention un procédé de détection de cellules transformées par une séquence déterminée d'intérêt comprenant:
a) la transformation des cellules avec un acide nucléique recombinant comprenant le gène d'intérêt et un gène de sélection comprenant une séquence de nucléotides codant pour un polypeptide à activité eutypine-réductase selon l'invention,
b) la mise en contact des cellules obtenues à l'étape a) avec un substrat de l'eutypine-réductase,
c) la détection d'une réaction de réduction du substrat,
d) le cas échéant, la sélection des cellules transformées donnant lieu à cette réaction de réduction.

L'invention a donc également pour objet un composé polypeptidique à activité eutypine-réductase, caractérisé en ce qu'il comprend la séquence d'acides aminés décrite à la figure 1.

Selon cette définition, l'activité eutypine-réductase observée est la capacité conférée chez l'hôte du parasite responsable de l'eutypiose, ou augmentée, par ce composé polypeptidique de dégrader ou de métaboliser l'eutypine sous la forme de produits de dégradation non toxiques pour l'hôte de référence normalement sensible à la toxicité liée à la présence d'eutypine.

L'eutypine ou hydroxy-4(méthyl-3-butène-3-ynyl-1)-3 benzaldéhyde est un acide faible lypophile dont on a mis en évidence qu'il pouvait être dégradé sous forme d'un dérivé hydroxylé, l'alcool hydroxy-4 (méthyl-3-butène-3-ynyl-1)-3 benzylique appelé eutypinol dans le cadre de la présente demande, grâce à une réaction enzymatique dont le constituant NADPH est un co-facteur spécifique lorsque la réaction est effectuée in vitro.

Le composé polypeptidique selon l'invention est donc capable, dans un environnement le permettant, de réaliser cette réaction enzymatique de métabolisation de l'eutypine sous la forme d'un dérivé non toxique, en particulier sous la forme d'eutypinol.

Le cas échéant l'activité eutypine-réductase peut être observée sur d'autres substrats aldéhydiques.

Le composé polypeptidique décrit précédemment est caractérisé par la présence dans sa structure d'une séquence d'acides aminés dont l'enchaînement a été donné à la figure 1 ; il peut le cas échéant comporter des groupements supplémentaires dont la présence et/ou la nature dépend par exemple de la cellule hôte dans laquelle il est exprimé.

Un composé avantageux selon l'invention est en outre caractérisé en ce qu'il a un poids moléculaire d'environ 36 kDa, calculé à partir de la séquence codante et mesuré à partir de la protéine purifiée.

Selon un mode de réalisation particulier de l'invention, le composé polypeptidique est caractérisé en ce qu'il est codé par une séquence de nucléotides comprenant l'enchaînement de nucléotides codant compris entre les nucléotides 22 et 175 (ces nucléotides extrêmes étant inclus) de l'enchaînement représenté à la figure 1.

L'invention a également pour objet des composés modifiés par rapport au composé polypeptidique précédemment défini. En particulier, il peut s'agir de composés polypeptidiques comprenant une séquence d'acides aminés dérivée de la séquence d'acides aminés décrite à la figure 1, par exemple délétion ou substitution d'au moins un résidu d'acide aminé contenu dans cette séquence, le composé formé ayant une activité eutypine-réductase selon la définition qui en est donnée ci-dessus.

Lorsque le composé de l'invention est obtenu par délétion d'au moins un résidu d'acides aminés de la séquence du composé polypeptidique décrit précédemment, il s'agit d'une délétion d'au moins un acide aminé non essentiel pour l'activité eutypine-réductase du composé répondant à la définition de la figure 1.

De même, lorsqu'on obtient un dérivé des composés ci-dessus décrits, par substitution de certains résidus d'acides aminés, il peut s'agir de les remplacer par des résidus ayant les mêmes caractéristiques.

Selon une variante de réalisation de l'invention, le composé polypeptique est caractérisé en ce qu'il est reconnu par des anticorps obtenus contre un polypeptide répondant à la séquence d'acides aminés représentée à la figure 1.

De tels anticorps sont par exemple des anticorps polyclonaux obtenus par immunisation d'un animal, notamment un lapin, avec le composé polypeptidique à activité eutypine-réductase selon l'invention et récupération des anticorps formés à partir du sérum du lapin.

Entre également dans le cadre de l'invention, un composé polypeptidique caractérisé en ce que sa séquence d'acides aminés présente une similitude au moins égale à 90% en nombre de résidus d'acides aminés, avec la séquence d'acides aminés représentée à la figure 1.

Par similitude, on entend l'identité des acides aminés comparés lorsque les deux séquences d'acides aminés sont comparées, ou en l'absence d'identité, la conservation des propriétés de l'acide aminé d'origine dans l'acide aminé substitué.

Les composés polypeptidiques selon l'invention peuvent être obtenus par toute méthode appropriée et en particulier peuvent être isolés à partir de végétaux, par exemple à partir du haricot *Vigna radiata* (Mung Bean) ou peuvent encore être synthétisés par voie chimique ou préparés par génie génétique notamment dans des cellules recombinantes.

D'autres végétaux peuvent également être utilisés pour extraire les composés de l'invention, tels que la vigne, la pomme, la carotte, etc...

L'invention vise également l'utilisation d'un composé polypeptidique à activité eutypine-réductase tel que défini ci-dessus ou d'une séquence nucléotidique codant un tel composé, pour lutter contre l'eutypiose chez les plantes ligneuses pérennes, notamment la vigne, l'abricotier, le cassissier, le cerisier, le tamaris, l'amandier ou le pommier. Elle porte également sur une plante produisant un composé polypeptidique à activité eutypine-réductase tel que défini plus haut, ladite plante étant choisie parmi la vigne, l'abricotier, le cassissier, le tamaris, l'amandier ou le pommier. En particulier, ledit composé produit par la plante est le produit d'un gène, exprimé sous le contrôle d'un promoteur hétérologue approprié. L'invention porte aussi sur les semences obtenues à partir de ces plantes.

D'autres caractéristiques et avantages apparaissent dans les figures dont la description suit, ainsi que dans les exemples.
Figure 1:Séquence nucléotidique de l'ADNc pleine longueur du gène VRER et séquence peptidique déduite

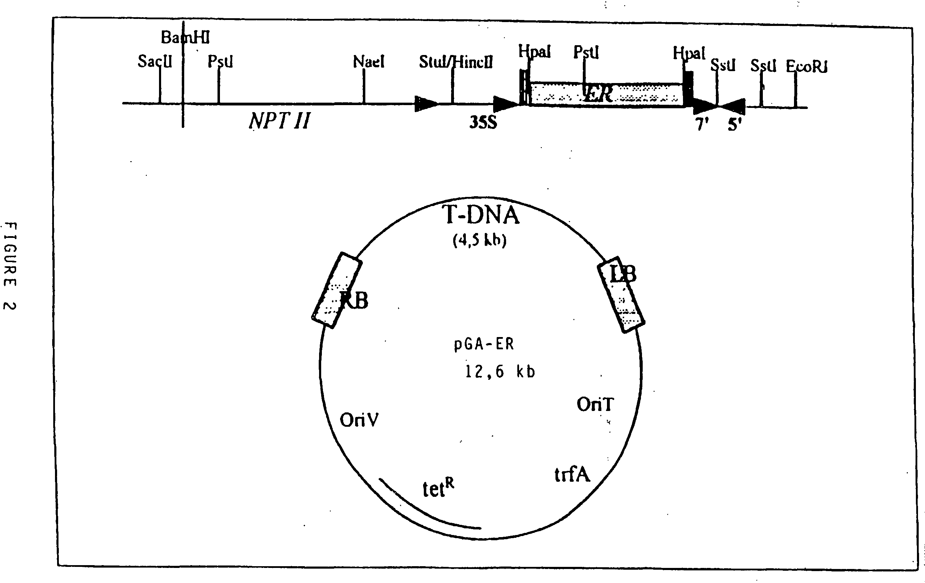

Figure 2:Représentation schématique du vecteur binaire pGA-ER. RB et LB : bordures droite (700 pb) et gauche (600 pb) du T-DNA ; OriV : origine de réplication (E. coli et Agrobacterium) ; OriT : origine de transfert conjugatif, trfA facteur de réplication « trans ». tet^{R} gène de résistance à la tétracycline ; NPTII : gène Néomycine PhosphoTransférase (résistance à la kanamycine) sous la dépendance du promoteur et du terminateur *nos* (nopaline synthase); 35S : promoteur fort du CaMV ; 5' et 7': terminateurs de transcription des gènes 5 et 7 de pTiA6 (plasmide Ti à octopine); ER : gène *VRER.*

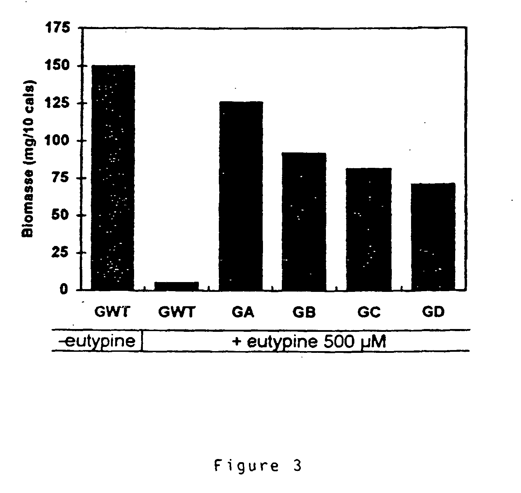

Figure 3: Effet de l'eutypine sur la croissance de cals de vigne. GWT - eutypine: cals non transformés cultivés en l'absence d'eutypine. GWT + eutypine: cals non transformés cultivés en présence de 500 µM d'eutypine. GA, GB, GC, GD + eutypine: cals transformés cultivés en présence de 500 µM d'eutypine.

### Partie expérimentale

### I- Mise en évidence d'une substance toxique synthétisée par Eutypa lata, l'eutypine. Etude de son mode d'action et de son devenir dans les cellules de vigne.

La localisation limitée du champignon dans le tronc et les bras des souches et la généralisation des symptômes dans tout l'appareil aérien suggèrent que le champignon agit à distance par l'intermédiaire d'une ou de plusieurs substances toxiques. Cette molécule a été isolée et identifiée, puis son mode d'action et son devenir ont été étudiés dans les cellules de vigne.

### Identification d'une substance toxique synthétisée par E lata

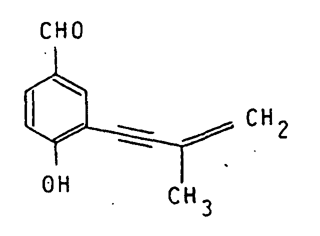

Parmi 15 métabolites secondaires synthétisés *in vitro* par E lata, et caractérisés chimiquement, une molécule toxique pour la vigne a été mise en évidence à l'aide de divers biotests. Il s'agit de l'hydroxy-4(méthyl-3-butène-3-ynyl-1-)-3 benzaldéhyde, appelée eutypine.

Plusieurs faits convergents ont permis de montrer que l'eutypine participe à l'expression des symptômes de l'eutypiose chez la vigne :
- l'eutypine s'avère toujours absente des ceps sains et se retrouve dans la sève, les feuilles, les tiges et les inflorescences des plantes infectées. Ce résultat a été obtenu par mise en oeuvre d'une chromatographie gazeuse couplée à une spectrophotométrie de masse en tandem (GC-SM-SM). Cependant, cette méthode d'identification n'a pas permis de doser l'eutypine présente dans les tissus de vigne;
- l'eutypine reproduit certains symptômes de la maladie lorsqu'elle est appliquée à des vitroplants de vigne ;
- l'eutypine induit des altérations ultrastructurales dans les feuilles de vitroplants sensiblement analogues à celles observées sur des plantes atteintes d'eutypiose : hypertrophie des chloroplastes, dilatation des thylakoïdes, rétraction du plasmalemme, lyse du cytoplasme, vésiculation des endomembranes conduisant à la destructuration totale des cellules des feuilles ;
- l'eutypine appliquée à des systèmes biologiques simplifiés provoque des symptômes plus graves chez les variétés sensibles à l'eutypiose que chez les cépages tolérants à cette maladie. Par exemple, les protoplastes obtenus à partir de feuilles de vigne de la variété Cabernet Sauvignon présentent une moindre tolérance à l'eutypine que les protoplastes de la variété Merlot.

L'ensemble des données obtenues conduit à la conclusion que l'eutypine est impliquée dans l'apparition des symptômes de la maladie. Au niveau de la plante, elle est synthétisée par le mycélium au printemps et migre, véhiculée par la sève, vers les organes herbacés La variation de la quantité d'eutypine élaborée pourrait expliquer, par exemple, le fait qu'une souche de vigne hébergeant le champignon parasite présente ou non des symptômes.

La recherche du mode d'action de l'eutypine dans les cellules de vigne a été permise grâce à la disponibilité de l'eutypine sous sa forme froide et sous une forme radioactive, marquée au carbone 14.

Il a été démontré que l'eutypine, acide faible lipophile, pénètre dans les cellules de vigne par diffusion passive. Cette molécule toxique s'accumule ensuite dans le cytoplasme par un mécanisme de piégeage d'acide et, en raison de son caractère lipophile, s'insère dans les lipides des membranes.

L'eutypine affecte le fonctionnement des systèmes membranaires des cellules de vigne. Elle provoque l'acidification du cytoplasme, la réduction du transport de leucine, l'activation à faible dose de la consommation d'oxygène et son inhibition au delà de 160 µM, et une diminution de la charge énergétique des cellules. Ces résultats suggèrent fortement que l'eutypine agit comme un transporteur mobile de protons (protonophore).

Le mode d'action de l'eutypine a été confirmé en recherchant ses effets sur le fonctionnement des mitochondries. L'eutypine agit comme un agent découplant de la phosphorylation oxydative. Le découplage complet des mitochondries est atteint pour des concentrations d'eutypine proches de 150 µM. L'effet découplant de l'eutypine est dû à un effet protonophore lié à la fonction alcool dissociable de la molécule puisqu'un analogue structural qui porte une fonction méthyle à la place de la fonction dissociable n'affecte que très faiblement le fonctionnement des mitochondries.

L'ensemble de ces données démontre que l'eutypine agit comme un transporteur mobile de protons. L'activité protonophore de l'eutypine peut, à elle seule, rendre compte des altérations physiologiques provoquées par le molécule toxique.

### Métabolisation de l'eutypine dans les cellules de vigne

Les cellules de vigne, placées en présence d'eutypine [C¹⁴]. produisent un composé radioactif identifié par spectographie de masse. Il s'agit d'un dérivé hydroxylé de l'eutypine, l'alcool hydroxy-4 (méthyl-3 ynyl-1)-3 benzylique, appelé eutypinol.

Ce composé ne montre pas de toxicité vis à vis de la vigne, même à des concentrations élevées, car il ne possède pas d'activité protonophore (Tableau I).

**Tableau 1**

| Effet de l'eutypinol sur la viabilité de protoplastes obtenus à partir de feuilles de vitropolants de Vitis vinifera cv. Cabernet Sauvignon. | | |
|---|---|---|
| **Traitement** | | **Viabilité après 48 heures** |
| Témoin | | 88 |
| Eutypine | | |
| | 100 µM | 67 |
| | 200 µM | 0 |
| Eutypinol | | |
| | 200 µM | 86 |
| | 500 µM | 83 |

Les protoplastes (4,5.10⁵/ml) sont cultivés en présence d'eutypine ou d'eutypinol pendant 48 heures

Afin de mettre en évidence une relation entre la capacité de métabolisation de l'eutypine des tissus de vigne et le comportement de différentes variétés de vigne vis à vis de l'eutypiose, les activités de détoxification des protoplastes de feuilles des cépages Ugni blanc (très sensible), Cabernet Sauvignon (sensible) et Merlot (tolérant) ont été comparées (Tableau I).

**Tableau II**

| Dégradation de l'eutypine par des protoplastes de différentes variétés de *Vitis vinifera* | | |
|---|---|---|
| Variétés | Sensibilité l'eutypiose au vignoble | Activité de détoxification (pmol/min/10⁵ cellules) |
| Ugni blanc | très sensible | 7,9 |
| Cabernet Sauvignon | sensible | 10,1 |
| Merlot | tolérant | 13,6 |

Les protoplastes (10⁶/ml) obtenus à partir de feuilles de vitroplants sont incubés pendant 1 heure à 30°C en présence d'eutypine (100 µM ; 0.4 kBq). Puis les composés phénylacétyléniques sont extraits, séparés par chromatographie sur couche mince et la vitesse de métabolisation de l'eutypine est calculée après détermination de la radioactivité associée à l'eutypine et à l'eutypinol.

Il apparaît que les protoplastes des variétés expérimentées peuvent transformer l'eutypine en eutypinol. La variété Merlot, considérée comme tolérante à l'eutypiose au vignoble, possède une activité de détoxication largement supérieure à celle de l'Ugni blanc, variété très sensible à la maladie. De plus, l'activité de détoxification de la variété Cabernet Sauvignon, cépage également sensible, est inférieure à celle de la variété Merlot.

Ces données montrent clairement l'existence d'une relation entre l'aptitude de ces cépages à métaboliser l'eutypine et leur comportement au vignoble à l'égard de l'eutypiose

La transformation de l'eutypine en eutypinol est catalysée par une enzyme non membranaire, nécessitant spécifiquement du NADPH, comme cofacteur. Son activité est réduite par des inhibiteurs des réductases tels que le disulfiram et l'acide parahydroxymercuribenzoïque, confirmant son appartenance à ce groupe d'enzymes Elle a été appelée « eutypine réductase ». Le schéma de la réaction est présenté ci-dessous.

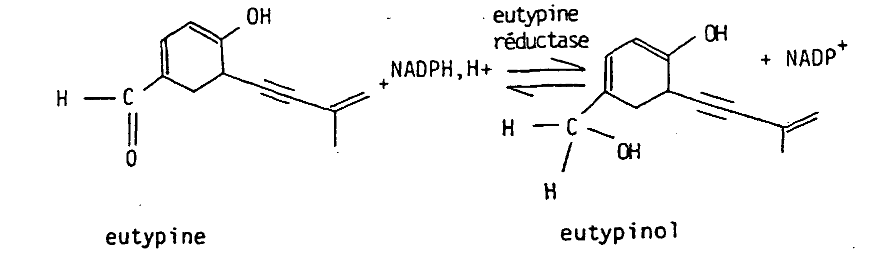

### II Recherche dans un système hétérologue d'un gène codant pour une protéine présentant une activité « eutypine réductase ».

L'objectif était d'isoler un gène codant pour une protéine présentant une activité « eutypine réductase ». Il est reconnu que la surexpression d'un gène d'une espèce donnée, dans cette même espèce, conduit souvent à une diminution globale de l'expression du gène endogène et du transgène. Par contre, la surexpression d'un gène hétérologue (appartenant à une espèce différente de celle qui est transformée) ne provoque pas ce phénomène.

Afin d'éviter le phénomène de co-suppression, on a recherché au sein d'espèces différentes de la vigne, la présence d'une enzyme capable de détoxifier l'eutypine. Parmi les espèces étudiées, Vigna radiata (VR) a présenté la plus forte capacité à réduire l'eutypine. L'isolement de la protéine a donc été réalisé à partir des tissus de cette espèce.

### Purification de la protéine ER

La protéine présentant une activité « eutypine réductase » a été purifiée à homogénéité selon le protocole suivant, puis séquencée.

Les graines de Mung Bean (Vigna radiata L.R. Wilcz) ont été imbibées une nuit dans de l'eau aérée Les graines ont ensuite été rincées avec de l'eau et semées sur de la vermiculite. Les plantules ont été récoltées après 4 jours de culture à 23°C et les hypocotyles (2 cm) ont été coupés pour l'extraction de l'enzyme

Cinq cents grammes d'hypocotyles de Mung Bean ont été broyés dans de l'azote liquide dans un broyeur à bille de type Dangoumeau Cent grammes de poudre obtenue ont été homogénéisés dans 2 volumes de tampon d'extraction composé de 0,1M de K-phosphate (pH 8.0), 10% de glycérol (p/v), 1% de polyvinylpyrrolidone M 40 000, 30 mM d'ascorbate de Na et de 5 mM de dithiothreitol (DTT). L'homogénat a été centrifugé 20 min à 48 000 g. Le surnageant ainsi obtenu a subi une double précipitation au sulfate d'ammonium entre 30 et 70% de saturation. Le culot issu de la précipitation à 70 % de sulfate d'ammonium a été dissout dans un petit volume de tampon 0,1 M K-phosphate (pH 8,0) contenant 10 % de glycérol et 1 mM de DTT. L'extrait enzymatique a été dessalé sur une colonne de Sephadex G25 (PD 10, Pharmacia), puis purifié en 6 étapes successives :
- chromatographie d'interaction hydrophobe sur une colonne de Phényl-Sepharose CL-4B (Pharmacia) ;
- chromatographie d'interaction ionique sur une colonne d'Hydroxylapatite (Econo-Pac HTP, BioRad) ;
- chromatographie d'exclusion moléculaire sur une colonne Superose 12 HR de type FPLC (Pharmacia) ; -
- chromatographie d'échange d'ions sur une colonne MonoQ 5/5 de type FPLC (Pharmacia) ;
- chromatographie par électrophorèse haute performance (HPEC) sur gel natif ;
- chromatographie par électrophorèse dénaturante (SDS-PAGE).

Alternativement, la protéine ER a pu être purifiée à partir de graines de Mung Bean, en appliquant la procédure suivante :

Cinq cents grammes d'hypocotyles de Mung Bean ont été broyés dans un broyeur à hélice en présence de deux volumes de tampon d'extraction composé de 0,1 M de tampon borate (p118.0), 10% de glycérol (p/v), 1% de polyvinylpyrrolidone 40 000, et de 4 mM de dithiothreitol (DTT). L'homogénat a été centrifugé 20 min à 48 000 g. Le surnageant ainsi obtenu a subi une double précipitation au sulfate d'ammonium entre 30 et 70 % de saturation. Le culot issu de la précipitation à 70% de sulfate d'ammonium a été dissout dans un faible volume de tampon 25 mM K-phosphate (pH 8,0) contenant 10 % de glycérol et 1 M de sulfate d'ammonium. L'extrait enzymatique a été purifié en 5 étapes successives :
- chromatographie d'interaction hydrophobe sur une colonne de Phényl-Sepharose CL-4B (Pharmacia);
- chromatographie d'interaction ionique sur une colonne d'Hydroxylapatite (Econo-Pac HTP, BioRad) ;
- chromatographie d'exclusion moléculaire sur une colonne Superose 12 HR de type FPLC (Pharmacia) ;
- chromatographie d'échange d'ions sur une colonne MonoQ 5/5 de type FPLC (Pharmacia)
- chromatographie par électrophorèse dénaturante (SDS-PAGE).

La protéine ainsi purifiée à homogénéité par SDS-PAGE a été hydrolysée pendant une nuit à 30°C par une endoprotéase-lysine et les peptides libérés ont été séparés par HPLC sur une colonne DEAE-C18 en phase reverse. Parmi les peptides séparés, cinq peptides ont été séquencés à l'aide d'un microséquenceur Applied Biosystem 470.

### Isolement et séquence nucléotidique du gène VRER

A l'aide d'amorces dégénérées issues de ces séquences peptidiques, un fragment d'ADN a été amplifié par la technique de la PCR (« polymerase chain reaction »). Une PCR inverse a ensuite permis d'isoler les parties 3' et 5' de ce clone. Enfin, le clone pleine longueur a été ensuite été isolé en choisissant les amorces appropriées aux extrémités 3' et 5' et a été séquencé plusieurs fois. L'ADN complémentaire présente un cadre de lecture ouvert de 975 nucléotides. La séquence en acides aminés déduite indique que le clone code pour un polypeptide composé de 325 acides aminés. La taille de la protéine est estimée à 36 Kd et a un Pi attendu de 6.34. La séquence du gène VRER présente des similitudes (entre 20 et 26% d'identité au niveau des acides aminés) avec les protéines de la famille des dihydrofalvonol réductases, et avec la HC toxine du maïs.

Afin de vérifier que la protéine codée par ce gène présente une activité « eutypine réductase » et est capable de réduire l'eutypine, cette protéine a été surexprimée dans un système bactérien, Escherichia coli, incapable à l'état naturel de réduire l'eutypine. Pour cela, l'ADN complémentaire du gène VRER a été cloné dans un vecteur d'expression, puis transféré dans les bactéries. Le lysat de ces bactéries qui surexpriment la protéine correspondant au gène VRER, utilise le cofacteur NADPH pour réduire l'eutypine. La production d'eutypinol a été mise en évidence par chromatographie en couche mince, d'un lysat bactérien incubé en présence d'eutypine radioactive. Cet ensemble de données montrent que la protéine correspondant au gène VRER présente in vitro une activité « eutypine réductase ».

### Caractéristiques biochimiques de la protéine codée par le gène VRER

La protéine codée par le gène VRER a été caractérisée biochimiquement. Elle présente un Km de 16.4 µM vis à vis de l'eutypine, ce qui représente une excellente affinité de cette enzyme pour le substrat eutypine. On a également recherché si cette protéine était capable de réduire d'autres substrats. La famille des benzaldehydes (benzaldehyde et certains de ses dérivés tels que methylbenzaldehyde, metoxybenzaldehyde, nitrobenzaldehyde, flurobenzaldehyde présentant un groupement en position meta et para), des aldéhydes linéaires tels que le decylaldéhyde ou l'hexanal, ou d'autres composés cycliques présentant une fonction aldéhyde comme le 4-pyridine carbaldéhyde ou le 2-fluraldéhyde sont également réduits par la protéine ER en présence de NADPH.

### III- Etude de l'expression du gène VRER dans les cellules de vigne

L'objectif de cette étude était de vérifier si l'expression du gène VRER, dans les cellules de vigne, est susceptible d'augmenter leur capacité à métaboliser l'eutypine en eutypinol. Pour cela, le gène VRER a été introduit dans un vecteur binaire de transformation, puis intégré dans des cals de vigne cv. Gamay. Les cals transformés ont été ensuite confrontés à l'eutypine pour analyser leur comportement et déterminer le niveau de résistance de la plante vis à vis de l'eutypine.

### Intégration du gène VRER dans un vecteur binaire de transformation

Le gène VRER a été intégré dans le plasmide binaire pGA (An G. et al, 1988, Binary vectors. In Plant Molecular Biology Manual A3. Kluwer Acad. Pub., Dordrecht, The Nederlands, 1-19), entre les bordures droites (RB) et les bordures gauches (LB). Ce vecteur binaire contient les gènes de résistance à la kanamycine et à la tétracycline. Le gène d'intérêt a été cloné dans le T-DNA, derrière le promoteur 35S du CaMV permettant son expression dans les cellules végétales.

La représentation schématique du vecteur binaire pGA-ER ainsi obtenu est donné à la figure 2.

Le plasmide pGA-ER a été introduit dans Agrobacterium tumefaciens souche C58. La présence de ce plasmide dans les bactéries transformées sur milieu sélectif (kanamycine 10 mg/l et tétracycline 5 mg/l), a été confirmée par analyse PCR. La souche C58 d'Agrobacterium tumefaciens contenant le plasmide pGA-ER est alors nommée AbC58-ER. Elle sera utilisée par la suite pour réaliser des expériences de transformation de cals de Vitis vinifera cv. Gamay.

### Transformation des cals de vigne via Agrobacterium tumefaciens

Les expériences de transformation ont été réalisées sur des cals de Vitis vinifera cv. Gamay, obtenus à partir de cellules de l'épiderme de baies.

La transformation génétique des cals de Vitis vinifera cv. Gamay a été effectuée via la souche d'Agrobacterium tumefaciens AbC58-ER, contenant le vecteur binaire pGA-ER.

Les cals, cultivés depuis 8 jours en boîte de Pétri de 55 mm (10 cals par boîte), ont été immergés dans la suspension bactérienne durant 20 minutes. L'inoculum a ensuite été retiré par aspiration et les cals ont été séchés 5 minutes sous la hotte. Ils ont ensuite été transférés sur un milieu neuf surmonté d'un disque de papier et mis en culture à l'obscurité à une température de 28°C. A l'issue d'une coculture de 48 heures, les cals ont été rincés délicatement avec du milieu de culture contenant 400 mg/l de carbénicilline (antibiotique destiné à éliminer les bactéries), séchés sur du papier buvard et transférés sur un milieu neuf avec 400 mg/l de carbénicilline. La pression de sélection a été appliquée 7 jours après inoculation : les cals ont été transférés sur un milieu « Gamay » sélectif contenant, en plus de la carbénicilline, un antibiotique sélectif (kanamycine à 75 mg/l).

Les cals ont été entretenus sur du milieu « Gamay », repiqués tous les 20 jours sur du milieu sélectif frais et placés dans une chambre de culture à 24°C, avec un éclairement de 100µmol.m⁻².sec⁻¹ et une photopériode de 16 heures.

Trois mois plus tard, après 4 repiquages successifs sur milieu sélectif neuf, des cals tolérants à la kanamycine ont été obtenus. Il ont ensuite été entretenus sur des milieux de culture sélectif renfermant 75 mg/l de kanamycine.

La vérification de l'intégration du gène VRER, dans le génome des cals de vigne manifestant une résistance marquée à la kanamycine, a été réalisée par la méthode de la PCR (Polymerase Chain Reaction), et par hybridation moléculaire (Southern blot). Enfin, le niveau d'expression du transgène a été déterminé par Western blot, à l'aide d'anticorps dirigés contre la protéine ER.

### Analyse du comportement des cals de vigne transformés vis à vis de l'eutypine

Les cals de vigne transformés génétiquement et exprimant le transgène VRER ont ensuite été confrontés à l'eutypine pour déterminer le niveau de résistance acquis par ces tissus vis à vis de l'eutypine.

Deux biotests ont été mis en oeuvre. Le premier (biotest 1) est fondé sur la confrontation de cellules de vigne cultivées en suspension avec de l'eutypine appliquée à différentes concentrations. Le second met en oeuvre des cals de vigne cultivés sur des milieux de culture renfermant de l'eutypine.

Dans le biotest 1, le pourcentage de cellules mortes a été estimé grâce au réactif à l'érythrosine. La concentration d'eutypine induisant un pourcentage de cellules mortes de 50% est déterminée chez la souche sauvage et chez les souches transformées. Les résultats présentés dans le tableau III montrent que les souches cellulaires de vigne transformées présentent un DL50 2 à 2,5 fois plus élevée que celle enregistrée pour la souche sauvage non transformée.

**Tableau III**

| Concentration d'eutypine provoquant la mortalité de 50% (DL50) des cellules de vigne transformées (GA, GB, GC, GD) ou non transformées (GWT), cultivées en suspension en présence de cette molécule toxique. | | | | | |
|---|---|---|---|---|---|
| Souches cellulaires de vigne | GWT | GA | GB | GC | GD |
| DL50 (µM d'eutypine) | 100 | 255 | 250 | 230 | 225 |
| GWT: souche sauvage, non transformée. GA, GB GC, GD: souches de vigne transformées. | | | | | |

Le biotest Il révèle que les cals de vigne transformés ont acquis un niveau de résistance à l'eutypine largement supérieur à la capacité des cals non transformés. Les résultats présentés ci-dessous montrent que les cals de la souche sauvage ne se développent pas en présence de 500µM d'eutypine et se nécrosent très rapidement. Par contre, la souche GA, cultivée en présence d'eutypine apportée à une concentration habituellement létale pour ces tissus, ne manifeste pas de nécroses et présente une croissance pratiquement similaire à celle des tissus témoins se développant en l'absence d'eutypine (Figure 3).

L'ensemble de ces résultats indique que l'introduction et l'expression du gène VRER dans les cellules de vigne confèrent une plus grande résistance à cette molécule toxique. Elle est due à la surproduction dans les cellules de vigne transformées de la protéine ER, capable de métaboliser l'eutypine en eutypinol, molécule non toxique pour la vigne données montrent l'efficacité de ce gène pour détoxiquer l'eutypine.

Les expériences de transformation ont été réalisées également sur des cals de *Vitis vinifera* cv. Ugni blanc en mettant en oeuvre le même protocole expérimental que celui utilisé pour la transformation des cals de *Vitis vinifera* cv. Gamay.

L'analyse du comportement des cals de *Vitis vinifera* cv. Ugni blanc vis à vis de l'eutypine a été réalisée en mettant en oeuvre le biotest II. Les résultats présentés ci-dessous montrent que les cals de vigne ont acquis un niveau de résistance à l'eutypine largement supérieur à celui des cals non transformés. En présence de 500 µM, les cals de la souche sauvage se nécrosent rapidement et ne se développent pas. A cette concentration, la croissance des cals de la souche UB transformée, n'est pratiquement pas affectée (Tableau IV). Ces résultats démontrent l'efficacité de ce gène pour détoxiquer l'eutypine dans les tissus de vigne.

**Tableau IV.**

| Développement des cals de *Vitis vinifera* cv. Ugni blanc non transformés (UBWT) et transformés (UBT) cultivés en présence de 500 µM d'eutypine. | |
|---|---|
| Souches cellulaires | Masse Moyenne d'un cal (mg) |
| UBWT | 25 |
| UBT | 150 |

### IV - Préparation d'anticorps dirigés contre la protéine codée par le gène VRER.

Pour obtenir des anticorps dirigés contre cette protéine, la démarche suivante a été mise en oeuvre.

### 1.1- Clonage du gène VRER.

Le fragment d'ADNc portant la partie active du gène *VRER* a été cloné dans le vecteur pT7.7 (Tabor S. et al, 1985, Proc. Natl. Acad. Sci. USA, 82, 1047-1078) aux sites EcoRI. Il a été ensuite introduit dans *E. coli.* Après avoir confirmé la bonne intégration du plasmide par Southern Blot, il a été vérifié que les souches de bactéries sélectionnées produisent la protéine ER capable de métaboliser l'eutypine en eutypinol.

### 1.2- Production et purification partielle de la protéine recombinante.

Les bactéries portant le transgène *VRER* ont été placées à 42°C pendant 20 minutes, puis la production de la protéine recombinante a été réalisée à 37°C pendant 1 heure. Après centrifugation, les bactéries ont été remises en suspension dans un Tampon Tris HCI 0,1 M (pH8,0), EDTA 1 mM, NaCI 0,1 M additionné de lyzozyme (1 mg/ml). Après incubation à 37°C pendant 15 minutes, la solution a été traitée par 0,2 mg/ml de Dnase en présence de PMSF (0,2 mM) et de MgCl₂ (4 mM) pendant 15 minutes. Après centrifugation à 12000 g, 10 minutes à 4°C, le culot protéique renfermant la protéine recombinante, a été lavé avec une solution d'urée 3M. Après centrifugation, l'extrait protéique a été dénaturé dans du tampon Tris Hcl 0,1 M (pH 7,5), contenant de l'urée (6M). Les protéines dénaturées ont ensuite été concentrées 5 fois (Centricon10).

### 1.3- Immunisation de lapins par l'extrait protéique renfermant la protéine recombinante.

Une fraction aliquote (50 µg) de l'extrait protéique renfermant la protéine recombinante a été fractionnée par électrophorèse préparative (gel polyacrylamide 12%, en présence de SDS). Les protéines ont été ensuite transférées sur une membrane de Nitrocellulose et la protéine recombinante a été repérée par coloration au bleu de Coomassie. Une bande de Nitrocellulose portant la protéine recombinante a été découpée et utilisée comme implant pour l'immunisation de deux lapins (Société Eurogentec). Quatre implantations ont été réalisées au cours des deux premiers mois d'immunisation et la saignée finale effectuée 1 mois après la dernière immunisation.

### 1.4- Purification partielle des anticorps dirigés contre la protéine ER

Les anticorps anti-protéine ER ont été partiellement purifiés par épuisement du sérum de lapins contre les protéines totales de E. coli. Les protéines extraites de E. coli sauvage sont déposées sur une membrane de Nitrocellulose, puis cette dernière est placée dans le sérum obtenu à partir des lapins. Cette opération est renouvelée plusieurs fois jusqu'à ce que le sérum soit épuisé.

Après vérification, le sérum ainsi obtenu est utilisé dans les analyses en Western blot.

## Revendications

1. Séquence de nucléotides, **caractérisée en ce qu'**elle code pour un polypeptide à activité eutypine-réductase, ce polypeptide comprenant la séquence d'acides aminés suivante:

2. Séquence de nucléotides selon la revendication 1, **caractérisée en ce qu'**elle comprend l'enchaînement de nucléotides suivant:

3. Séquence de nucléotides selon la revendication 2, **caractérisée en ce qu'**elle est constituée par l'enchainement d'ADNc suivant:

4. Séquence de nucléotides **caractérisée en ce qu'**elle code pour un polypeptide à activité eutypine-réductase et **en ce qu'**elle présente une similitude en nombre de nucléotides, supérieure ou égale à 90%, avec une séquence de nucléotides selon la revendication 2 ou la revendication 3.

5. Séquence de nucléotides selon l'une quelconque des revendications 1 à 4, ou séquence de nucléotides hybridant dans des conditions de forte stringence avec une séquence de nucléotides selon l'une quelconque des revendications 1 à 4, les conditions de forte stringence étant une hybridation dans une solution SSC (2X) 1% SDS 5 minutes à 25° et 2 fois 15 minutes à 45°C et un lavage dans une solution SSC (0,2X), 1 % SDS 2 fois 15 minutes à 45°C, ou séquence de nucléotides présentant une similitude en nombre de nucléotides supérieure ou égale à 75% avec une séquence selon l'une quelconque des revendications 1 à 4, **caractérisée en ce qu'**elle code pour un polypeptide à activité eutypine-réductase et **en ce qu'**elle est placée sous le contrôle d'un promoteur approprié pour l'expression hétérologue.

6. Séquence de nucléotides selon l'une quelconque des revendications 1 à 5, **caractérisée en ce qu'**elle est contenue dans un vecteur d'expression et/ou de clonage.

7. Séquence de nucléotides selon l'une quelconque des revendications 1 à 6, **caractérisée en ce qu'**elle est contenue dans le vecteur binaire pGA.

8. Séquence de nucléotides selon l'une quelconque des revendications 1 à 7, **caractérisée en ce qu'**elle est placée sous le contrôle d'un promoteur d'expression approprié pour l'expression dans des cellules procaryotes et/ou eucaryotes.

9. Séquence de nucléotides selon la revendication 8, **caractérisée en ce que** le promoteur d'expression est un promoteur hétérologue.

10. Séquence de nucléotides selon l'une des revendications 1 à 9, **caractérisée en ce qu'**il s'agit d'une séquence d'ADNc.

11. Cellule recombinante procaryote **caractérisée en ce qu'**elle contient une séquence de nucléotides selon l'une quelconque des revendications 1 à 10.

12. Cellule recombinante selon la revendication 11, **caractérisée en ce qu'**il s'agit d'une cellule de bactérie, par exemple d'une cellule de Escherichia coli ou d'Agrobacterium tumefaciens.

13. Cellule eucaryote recombinée avec une séquence de nucléotides selon la revendication 9 ou 10.

14. Cellule eucaryote recombinante selon la revendication 13, **caractérisée en ce qu'**il s'agit d'une cellule végétale.

15. Cellule recombinante selon la revendication 13, **caractérisée en ce qu'**il s'agit d'une cellule de semence de plante.

16. Cellule recombinante selon l'une quelconque des revendications 13 à 15, **caractérisée en ce qu'**il s'agit d'une cellule de vigne.

17. Plante recombinante ayant intégré dans son génome une séquence de nucléotides selon la revendication 9 ou 10.

18. Composé polypeptidique à activité eutypine-réductase, **caractérisé en ce qu'**il comprend la séquence d'acides aminés suivante:

19. Composé polypeptidique selon la revendication 18, **caractérisé en ce qu'**il a un poids moléculaire d'environ 36 kDa.

20. Composé polypeptidique selon l'une quelconque des revendications 18 ou 19, **caractérisé en ce qu'**il est codé par une séquence de nucléotides comprenant l'enchaînement de nucléotides codant suivant :

21. Composé polypeptidique, **caractérisé en ce que** sa séquence d'acides aminés est dérivée de la séquence d'acides aminés du composé selon la revendication 18, par délétion d'au moins un acide aminé non nécessaire pour l'activité eutypine-réductase ou substitution d'au moins un résidu d'acide aminé par des résidus ayant les mêmes caractéristiques, le composé formé ayant une activité eutypine-réductase.

22. Composé polypeptidique selon l'une quelconque des revendications 18 à 21, **caractérisé en ce qu'**il est reconnu par des anticorps obtenus contre un polypeptide répondant à la séquence d'acides aminés suivante:

23. Composé polypeptidique à activité eutypine-réductase, **caractérisé en ce que** sa séquence d'acides aminés présente une similitude au moins égale à 90% en nombre de résidus d'acides aminés, avec la séquence d'acides aminés du composé polypeptidique selon la revendication 18.

24. Utilisation d'une séquence de nucléotides selon l'une quelconque des revendications 1 à 10 ou d'un composé polypeptidique à activité eutypine-réductase selon l'une quelconque des revendications 18 à 23, pour lutter contre l'eutypiose chez les plantes ligneuses pérennes, notamment la vigne, l'abricotier, le cassissier, le cerisier, le tamaris, l'amandier ou le pommier.

25. Plante selon la revendication 17, choisi parmi la vigne, l'abricotier, le cassissier, le cerisier, le tamaris, l'amandier ou le pommier.

26. Plante produisant un composé polypeptidique selon l'une quelconque des revendications 18 à 23, ledit composé étant le produit d'un gène, sous le contrôle d'un promoteur hétérologue approprié.

27. Plante selon la revendication 26, **caractérisée en ce qu'**il s'agit de la vigne.

28. Semences obtenues à partir des plantes selon la revendication 25 à 27.

29. Procédé de production de plantes ou de semences exprimant un composé polypeptidique à activité eutypine-réductase, **caractérisé en ce qu'**il comprend les étapes de :
a) transformation d'une cellule végétale, avec une séquence de nucléotides selon l'une quelconque des revendications 1 à 8, dans des conditions permettant l'expression de façon stable et fonctionnelle de la protéine à activité eutypine-réductase codée par la susdite séquence de nucléotides;
b) régénération de plantes à partir de la cellule de plante transformée de l'étape a), pour obtenir des plantes exprimant la protéine à activité eutypine-réductase,
c) le cas échéant, obtention de semences à partir des plantes modifiées obtenues à l'étape b).

30. Procédé selon la revendication 29, **caractérisée en ce que** la plante est la vigne.

31. Utilisation d'une séquence de nucléotides selon l'une quelconque des revendications 1 à 10, comme agent de sélection pour la transformation de cellules.

32. Acide nucléique recombinant, **caractérisé en ce qu'**il contient un agent de sélection selon la revendication 31, en association avec une séquence d'intérêt, ladite association permettant l'intégration conjointe du marqueur de sélection et de la séquence d'intérêt, dans un hôte cellulaire déterminé.

33. Procédé de détection de cellules transformées par une séquence déterminée d'intérêt comprenant :
a) la transformation des cellules avec un acide nucléique selon la revendication 32,
b) la mise en contact des cellules obtenues à l'étape a) avec un substrat de l'eutypine-réductase,
c) la détection d'une réaction de réduction du substrat,
d) le cas échéant la sélection des cellules transformées donnant lieu à cette réaction de réduction.

## Patentansprüche

1. Nukleotidsequenz, **dadurch gekennzeichnet, dass** diese für ein Polypeptid mit Eutypin-Reduktase-Aktivität kodiert, wobei das Polypeptid die folgende Aminosäuresequenz aufweist:

2. Nukleotidsequenz gemäß Anspruch 1, **dadurch gekennzeichnet, dass** diese die folgende Nukleotidkette aufweist:

3. Nukleotidsequenz gemäß Anspruch 2, **dadurch gekennzeichnet, dass** diese durch die folgende cDNA-Kette gebildet ist:

4. Nukleotidsequenz, **dadurch gekennzeichnet, dass** diese für ein Polypeptid mit Eutypin-Reduktase-Aktivität kodiert, und **dadurch**, dass diese eine Ähnlichkeit in der Anzahl der Nukleotide mit einer Nukleotidsequenz gemäß Anspruch 2 oder Anspruch 3 von mehr als oder gleich 90% aufweist.

5. Nukleotidsequenz gemäß einem der Ansprüche 1 bis 4, oder Nukleotidsequenz, welche unter stark stringenten Bedingungen mit einer Nukleotidsequenz gemäß einem der Ansprüche 1 bis 4 hybridisiert, wobei die stark stringenten Bedingungen eine Hybridisierung darstellen in einer Lösung SSC (2X) 1 % SDS 5 Minuten bei 25° und 2 Mal 15 Minuten bei 45° sowie eine Waschung in einer Lösung SSC (0,2X), 1% SDS 2 Mal 15 Minuten bei 45°C, oder Nukleotidsequenz, welche eine Ähnlichkeit in der Anzahl der Nukleotide mit einer Sequenz gemäß einem der Ansprüche 1 bis 4 von mehr als oder gleich 75% aufweist, **dadurch gekennzeichnet, dass** sie für ein Polypeptid mit Eutypin-Reduktase-Aktivität kodiert und **dadurch**, dass sie unter Kontrolle eines für die heterologe Expression geeigneten Promotors ist.

6. Nukleotidsequenz gemäß einem der Ansprüche 1 bis 5, **dadurch gekennzeichnet, dass** sie in einem Vektor der Expression und/oder des Klonierens enthalten ist.

7. Nukleotidsequenz gemäß einem der Ansprüche 1 bis 6, **dadurch gekennzeichnet, dass** sie in dem pGA-Binärvektor enthalten ist.

8. Nukleotidsequenz gemäß einem der Ansprüche 1 bis 7, **dadurch gekennzeichnet, dass** sie unter Kontrolle eines Promotors für die Expression ist, der für die Expression in prokaryotische und/oder eukaryotische Zellen geeignet ist.

9. Nukleotidsequenz gemäß Anspruch 8, **dadurch gekennzeichnet, dass** der Promotor für die Expression ein heterologer Promotor ist.

10. Nukleotidsequenz gemäß einem der Ansprüche 1 bis 9, **dadurch gekennzeichnet, dass** es sich um eine cDNA-Sequenz handelt.

11. Rekombinante prokaryotische Zelle, **dadurch gekennzeichnet, dass** sie eine Nukleotidsequenz gemäß einem der Ansprüche 1 bis 10 enthält.

12. Rekombinante Zelle gemäß Anspruch 11, **dadurch gekennzeichnet, dass** es sich um die Zelle eines Bakteriums handelt, zum Beispiel um eine Zelle von Escherichia coli oder Agrobacterium tumefaciens.

13. Eukaryotische Zelle, die mit einer Nukleotidsequenz gemäß Anspruch 9 oder 10 rekombiniert ist.

14. Rekombinante eukaryotische Zelle gemäß Anspruch 13, **dadurch gekennzeichnet, dass** es sich um eine pflanzliche Zelle handelt.

15. Rekombinante Zelle gemäß Anspruch 13, **dadurch gekennzeichnet, dass** es sich um eine Pflanzensamenzelle handelt.

16. Rekombinante Zelle gemäß einem der Ansprüche 13 bis 15, **dadurch gekennzeichnet, dass** es sich um eine Zelle einer Weinrebe handelt.

17. Rekombinante Pflanze, in deren Genom eine Nukleotidsequenz gemäß Anspruch 9 oder 10 eingebaut ist.

18. Polypeptidzusammensetzung mit Eutypin-Reduktase-Aktivität, **dadurch gekennzeichnet, dass** sie die folgende Aminosäuresequenz enthält:

19. Polypeptidzusammensetzung gemäß Anspruch 18, **dadurch gekennzeichnet, dass** sie ein Molekulargewicht von ungefähr 36 kDa aufweist.

20. Polypeptidzusammensetzung gemäß einem der Ansprüche 18 oder 19, **dadurch gekennzeichnet, dass** sie für eine Nukleotidsequenz kodiert ist, welche die folgende kodierende Nukleotidkette aufweist:

21. Polypeptidzusammensetzung, **dadurch gekennzeichnet, dass** deren Aminosäuresequenz von der Aminosäuresequenz der Zusammensetzung gemäß Anspruch 18 abgeleitet wird durch Deletion wenigstens einer Aminosäure, die für die Eutypin-Reduktase-Aktivität nicht erforderlich ist, oder Substitution wenigstens eines Aminosäurerests durch Reste, welche die gleichen charakteristischen Eigenschaften aufweisen, wobei die gebildete Zusammensetzung eine Eutypin-Reduktase-Aktivität aufweist.

22. Polypeptidzusammensetzung gemäß einem der Ansprüche 18 bis 21, **dadurch gekennzeichnet, dass** diese von Antikörpern wiedererkannt wird, die gegen ein Polypeptid erhalten wurden, das auf die folgende Aminosäuresequenz reagiert:

23. Polypeptidzusammensetzung mit Eutypin-Reduktase-Aktivität, **dadurch gekennzeichnet, dass** deren Aminosäuresequenz eine Ähnlichkeit von wenigstens 90% in der Anzahl der Aminosäurereste mit der Aminosäuresequenz der Polypeptidzusammensetzung gemäß Anspruch 18 aufweist.

24. Verwendung einer Nukleotidsequenz gemäß einem der Ansprüche 1 bis 10 oder einer Polypeptidzusammensetzung mit Eutypin-Reduktase-Aktivität gemäß einem der Ansprüche 18 bis 23, um Eutypiose bei perennierenden holzigen Pflanzen, insbesondere Weinrebe, Aprikosenbaum, Johannisbeerstrauch, Kirschbaum, Tamariske, Mandelbaum oder Apfelbaum, zu bekämpfen.

25. Pflanze gemäß Anspruch 17, welche aus Weinrebe, Aprikosenbaum, Johannisbeerstrauch, Kirschbaum, Tamariske, Mandelbaum oder Apfelbaum gewählt ist.

26. Pflanze, welche eine Poiypeptidzusammensetzung gemäß einem der Ansprüche 18 bis 23 produziert, wobei die Zusammensetzung das Produkt eines Gens unter Kontrolle eines geeigneten heterologen Promotors ist.

27. Pflanze gemäß Anspruch 26, **dadurch gekennzeichnet, dass** es sich um die Weinrebe handelt.

28. Samen, die aus Pflanzen gemäß Anspruch 25 bis 27 erhalten werden.

29. Verfahren zur Herstellung von Pflanzen oder Samen, welche eine Polypeptidzusammensetzung mit Eutypin-Reduktase-Aktivität exprimieren, **dadurch gekennzeichnet, dass** es die folgenden Schritte aufweist:
a) Transformation einer pflanzlichen Zelle mit einer Nukleotidsequenz gemäß einem der Ansprüche 1 bis 8 unter Bedingungen, die eine stabile und wirksame Expression des Proteins mit Eutypin-Reduktase-Aktivität, kodiert durch die oben genannte Nukleotidsequenz, ermöglichen;
b) Regeneration von Pflanzen ausgehend von der in Schritt a) transformierten pflanzlichen Zelle, um Pflanzen zu erhalten, welche das Protein mit Eutypin-Reduktase-Aktivität exprimieren,
c) gegebenenfalls Erhalten von Samen von im Schritt b) erhaltenen modifizierten Pflanzen.

30. Verfahren gemäß Anspruch 29, **dadurch gekennzeichnet, dass** es sich um die Weinrebe handelt.

31. Verwendung einer Nukleotidsequenz gemäß einem der Ansprüche 1 bis 10 als Selektionsmittel für die Transformation von Zellen.

32. Rekombinante Nukleinsäure, **dadurch gekennzeichnet, dass** diese ein Selektionsmittel gemäß Anspruch 31 enthält in Verbindung mit einer Sequenz von Interesse, wobei die Verbindung den gemeinsamen Einbau des Selektionsmarkers und der Sequenz von Interesse in eine bestimmte Wirtszelle ermöglicht.

33. Verfahren zur Detektion von durch eine bestimmte Sequenz von Interesse transformierte Zellen, welches aufweist:
a) die Transformation von Zellen mit einer Nukleotidsequenz gemäß Anspruch 32,
b) das In-Kontakt-Bringen von in Schritt a) erhaltenen Zellen mit einem Substrat der Eutypin-Reduktase,
c) die Detektion einer Reduktionsreaktion des Substrats,
d) gegebenenfalls Selektion von transformierten Zellen, welche einen Ort für diese Reduktionsreaktion bieten.

## Claims

1. Nucleotide sequence, **characterized in that** it codes for a polypeptide with eutypine reductase activity, this polypeptide comprising the following amino acid sequence:

2. Nucleotide sequence according to Claim 1, **characterized in that** it comprises the following nucleotide sequence:

3. Nucleotide sequence according to Claim 2, **characterized in that** it is constituted of the following cDNA sequence :

4. Nucleotide sequence **characterized in that** it encodes a polypeptide having eutypine - reductase activity and it exhibits a homology greater than or equal to 90% based on the number of nucleotides, with a nucleotide sequence according to Claim 2 or Claim 3.

5. Nucleotide sequence according to any one of the Claims 1 to 4, or nucleotide sequence hybridizing under highly stringent conditions with a nucleotide sequence according to any one of the Claims 1 to 4, the high stringency conditions being hybridization in a solution of SSC (2x) 1% SDS 5 min. at 25°C and 2 times 15 min at 45°C or nucleotide sequence exhibiting a sequence homology greater than or equal to 75% based on the number of nucleotides with a sequence according to any one of the Claims 1 to 4, **characterized in that** it is placed under the control of a promoter suitable for heterologous expression.

6. Nucleotide sequence according to any one of the Claims 1 to 6, **characterized in that** it is contained in an expression and/or cloning vector.

7. Nucleotide sequence according to any one of the Claims 1 to 6, **characterized in that** it is contained in the binary vector pGA.

8. Nucleotide sequence according to any one of the Claims 1 to 7, **characterized in that** it is placed under the control of an expression promoter suitable for expression in prokaryotic and/or eukaryotic cells.

9. Nucleotide sequence according to claim 8, **characterized in that** the expression promoter is a heterologous promoter.

10. Nucleotide sequence according to anyone of claims 1 to 9, **characterized in that** it is a cDNA sequence.

11. Recombinant prokaryotic cell **characterized in that** it contains a nucleotide sequence according to any one of the Claims 1 to 10.

12. Recombinant cell according to Claim 11, **characterized in that** it is a bacterial cell, for example a cell of E. coli or Agrobacterium tumefaciens.

13. Eukaryotic cell recombined with a nucleotide sequence according to claim 9 or 10.

14. Recombinant cell according to Claim 13, **characterized in that** it is a plant cell.

15. Recombinant cell according to Claim 13, **characterized in that** it is a plant seed cell.

16. Recombinant cell according to any one of the Claims 13 to 15, **characterized in that** it is a grapevine cell.

17. Recombinant plant which has integrated into its genome a nucleotide sequence according to any one of the claims 9 or 10.

18. Polypeptide compound with eutypine reductase activity, **characterized in that** it comprises the following amino acid sequence:

19. Polypeptide compound according to Claim 18, **characterized in that** it has a molecular weight of about 36 kDa.

20. Polypeptide compound according to any one of the Claims 18 or 19, **characterized in that** it is encoded by a nucleotide sequence comprising the following coding nucleotide sequence:

21. Polypeptide compound, **characterized in that** its amino acid sequence is derived from the amino acid sequence according to Claim 18 by deletion of at least one amino acid residue which is not necessary for the eutypine reductase activity, or substitution of at least one amino acid residue having the same characteristics, the compound formed having a eutypine reductase activity.

22. Polypeptide compound according to any one of the Claims 18 to 21, **characterized in that** it is recognized by antibodies obtained against a polypeptide corresponding to the following amino acid sequence:

23. Polypeptide compound with eutypine reductase activity, **characterized in that** its amino acid sequence exhibits a homology at least equal to 90% of the number of amino acid residues with the amino acid sequence of the polypeptide compound according to Claim 18.

24. Use of a nucleotide sequence according to anyone of claims 1 to 10 or of a polypeptide compound with eutypine reductase activity according to anyone of claims 18 to 23 to combat eutypa dieback in perennial ligneous plants, in particular the grapevine, the apricot, the black currant, the cherry, the tamarind, the almond or the apple.

25. Plant according to Claim 17, selected among the grapevine, the apricot, the black currant, the cherry, the tamarind, the almond or the apple.

26. Plant producing a polypeptide compound according to any one of the Claims 18 to 23, said compound being the product of a gene, under the control of a heterologous promoter.

27. Plant according to Claim 26, **characterized in that** it is the grapevine.

28. Seeds obtained from the plants according to Claims 25 to 27.

29. Production process for plants or seeds expressing a polypeptide compound with eutypine reductase activity, **characterized in that** it comprises the steps of:
a) transformation of a plant cell with a nucleotide sequence according to any one of the Claims 1 to 8, under conditions permitting the functional and stable expression of the protein with eutypine reductase activity encoded by the above-mentioned sequence of nucleotides;
b) regeneration of plants from the transformed plant cell of step a) to obtain plants expressing the protein with eutypine reductase activity,
c) where appropriate, production of seeds from the modified plants obtained in step b).

30. Process according to Claim 29, **characterized in that** the plant is the grapevine.

31. Use of a nucleotide sequence according to any one of the Claims 1 to 10, as selection agent for the transformation of cells.

32. Recombinant nucleic acid, **characterized in that** it contains a selection agent according to Claim 31, in association with a sequence of interest, said association permitting the conjugate integration of the selection marker and the sequence of interest in a defined cell host.

33. Detection procedure for cells transformed by a defined sequence of interest comprising:
a) the transformation of the cells with a nucleic acid according to Claim 32,
b) the placing of the cells obtained in step a) in contact with a substrate for the eutypine reductase
c) the detection of a reduction reaction undergone by the substrate,
d) where appropriate, the selection of the transformed cells giving rise to this reduction reaction.
